# EUROPEAN PATENT SPECIFICATION

(11) **EP 1 716 805 B1**
(45) Date of publication and mention of the grant of the patent: **16.03.2011**
(21) Application number: 06250192.9
(22) Date of filing: 13.01.2006
(51) Int. Cl.: A61B 5/00

(54) **Wireless heart rate monitoring system**
Drahtloses Herzratenmonitor-System
moniteur de la fréquence cardiaque sans fils

(30) Priority: 29.04.2005 US 117484
(43) Date of publication of application: 02.11.2006
(60) Divisional application: 10013165.5
(73) Proprietor: IDT Technology Limited, Kowloon, Hong Kong SAR (CN)
(72) Inventor: Chan, Raymond, Hunghom Kowloon Hong Kong SAR (CN)
(74) Representative: Martin, David John

(56) References cited:
- EP-A- 0 836 165
- US-A1- 2003 040 305
- US-A1- 2003 073 911

## Description

The present invention relates to a heart rate monitoring system.

### BACKGROUND OF THE INVENTION

More particularly, although not exclusively, the invention is concerned with a wireless-transmission heart rate monitor comprising a body-mounted heart beat detector and a remote receiver/display unit which might also be worn on the body, for example like a watch about the wearer's wrist.

Conventional heart rate monitors include a body-mounted detector such as a chest belt which transmits a signal to the remote display unit upon detection of each heart beat. The remote display unit receives such signals and then calculates an ECG (electrocardiogram) pulse count for display purposes. Such apparatus are generally reliable in environments free of interference that may be caused by radiation from nearby transmitting devices such as mobile phones or power lines. This problem has been addressed to in US Patent Nos. 5,611,346 and 5,632,279.

In the former document, two identification pulses are generated by each body-mounted transmitter whereby the timing of all between the identification pulses corresponds to a specific time interval determined to reach transmitter-receiver pair, on the basis of which specific interval the receiver identifies the measuring pulses intended for it. In the latter document, heart beat signals (bursts) are amplitude-modulated with a frequency or a frequency sequence characteristic of the transmitter/receiver unit, on the basis of which the receiver recognises the bursts only intended for it.

In the known prior art, heart rate monitors including those disclosed in the above referenced US patents, the actual heart rate data (typically in beats per minute) to be displayed is not computed within the body-mounted detector unit, but rather at a remote display location for example by a wrist-watch-type main unit worn on the wrist. Moreover, the wireless signals transmitted by the transmitter to the receiver are in the form of ECG bursts to be computed at the receiving end of the wireless transmission so to speak. Accordingly, complex and often unreliable methods must be employed in an attempt to distinguish one transmission from another where multiple users - say in a hospital or sporting environment - are wearing body-mounted transmitters in local proximity to one another.

US 2003/0073911 A1 describes a pace control device comprising a chest belt sensor device and a data receiver. The data receiver obtains the data on the heart rate and the workout paces and displays the information on the monitor so that the user can adjust their working at an optimum level.

US 2003/0040305 describes a wireless, programmable system according to the first part of claim 1, for bio-potential signal acquisition, including a base unit and a plurality of individual wireless, remotely programmable transceivers that connect to patch electrodes. The base unit manages the transceivers by issuing wireless commands.

EP 0 836 165 describes a telemetric measuring method and system, in which telemetric data is transmitted from a sensor to a receiver with pulse groups, the combination of which is specific to a particular measure variable.

### OBJECTS OF THE INTENTION

It is an object of the present invention to overcome or substantially ameliorate the above disadvantages and/or more generally to provide an improved wireless heart rate monitor.

### SUMMARY OF THE INVENTION

According to the invention, there is provided a wireless heart rate monitoring system comprising a detection unit and a display unit, the two units being separate and cooperatively in communication via a wireless link. The detection unit comprises a casing, attaching means at the casing for attaching the casing on a user, a heart rate detector at the casing for detecting ECG pulses of said user, a microprocessor in the casing for computing a heart rate based on the ECG pulses detected by the heart rate detector, and a transmitter at the casing for transmitting a wireless signal indicative of the heart rate computed by the microprocessor to the display unit. The display unit comprises a casing, a receiver at the casing for receiving said wireless signal from the detection unit, and a display at the casing for displaying the heart rate received by the receiver,
the detection unit includes timing means for controlling its transmitter to transmit said wireless signal at a different time from that of another detection unit nearby,
characterised in that the transmitter is controlled by the microprocessor to transmit said wireless signal at substantially fixed intervals, and the timing means comprises a series of time slots divided from said fixed interval, one of which time slots is allocated for the transmitter to transmit said wireless signal, and that the timing means is associated with a button or key for manual operation to change the transmission time slot, whereby successive depression of the button or key shifts the time of transmission of said wireless signal from one time slot to the next until a vacant time slot is reached.

Preferably, the attaching means comprises a strap or straps for attaching around the chest of said user.

Preferably, the display unit is of a wrist-mounted type.

It is preferred that the transmitter comprises a radio frequency transmitter and the receiver comprises a radio frequency receiver.

It is preferred that the transmitter is controlled by the microprocessor to transmit said wireless signal at substantially fixed intervals.

In a preferred embodiment, said wireless signal comprises a coded data packet that includes data representing the heart rate computed by the microprocessor.

More preferably, the coded data packet further includes a code identifying the detection unit.

### BRIEF DESCRIPTION OF DRAWINGS

The invention will now be more particularly described, by way of example only, with reference to the accompanying drawings, in which:
Figure 1 is a front view of an embodiment of a wireless heart rate monitoring system in accordance with the invention, comprising a chest-belt detection unit and a wrist-mounted display unit;
Figure 2 is a schematic functional block diagram of the transmitting and receiving units of Figure 1;
Figures 3A to 3C are schematic waveform diagrams showing comparison of transmission protocol between the present invention and the prior art; and
Figures 4A to 4C are schematic waveform diagrams showing signals transmitted by the detection unit to the display unit.

### DETAILED DESCRIPTION OF PREFERRED EMBODIMENT

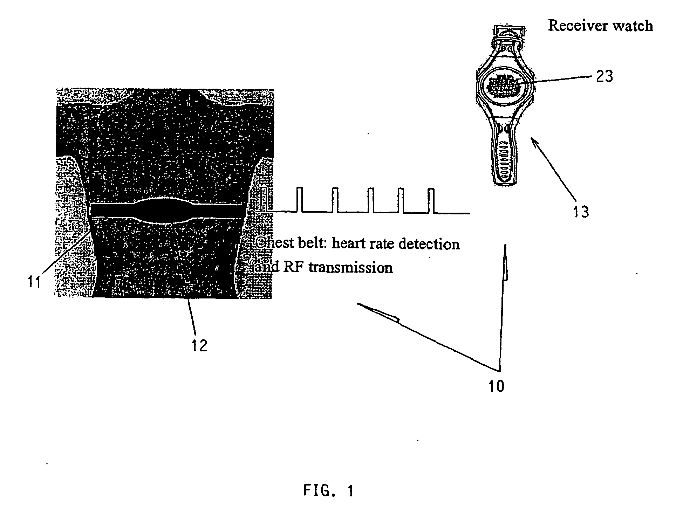

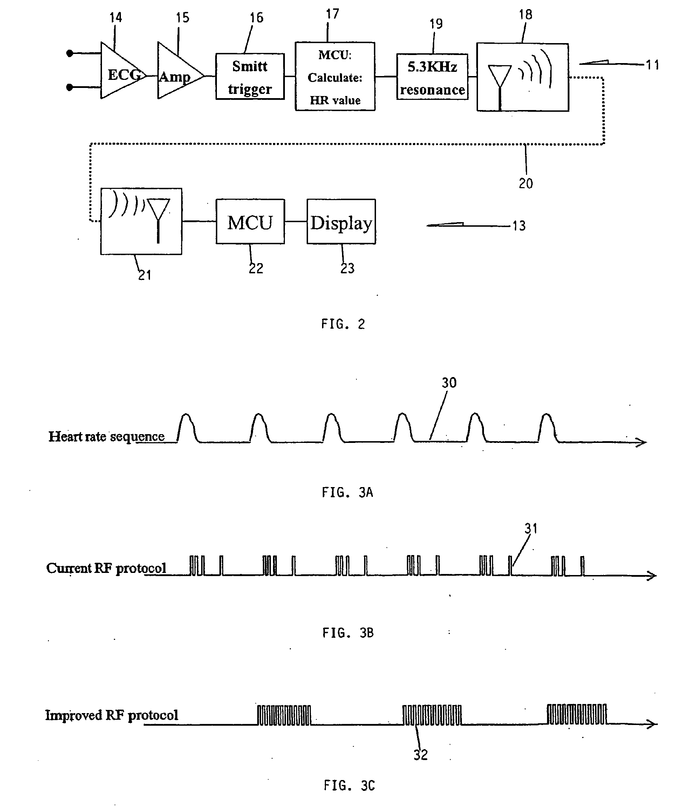

Referring initially to Figures 1 and 2 of the drawings, there is shown a wireless heart rate monitoring system 10 embodying the invention, which comprises two separate units i.e. a detection unit 11 and a display unit 13 that are in communication with each other via a wireless link 20 for operation.

The detection unit 11 is in the form of a chest belt, including a strap or straps for attaching around the chest of a user for detection of his/her heartbeat. The display unit 13 resembles a wrist-watch which has an LCD display panel 23 on its casing for displaying a measured heart rate.

The detection unit 11 has a casing 12 and a heart rate detector 14 supported thereby on the rear side for bearing against the user's chest to detect his/her heartbeat. Housed in the casing 12 and connected in series from the detector 14, there are an amplifier 15, a Smitt trigger 16, an MCU (microprocessor control unit) 17, a 5.3kHz modulator 19 and an RF (radio frequency) transmitter 18.

The detected heartbeat, an ECG signal, will be amplified by the amplifier 15 and then converted by the Smitt trigger 16 into pulses for subsequent processing by the MCU 17. Compared with general triggers, the Smitt trigger 16 offers the advantage of better control over false trigger signals as may be caused by noises.

The MCU 17 is programmed, inter alia, to calculate a heart rate (beats per minute) based on the heartbeat pulses input by the Smitt trigger 16 and then to transform the calculated heart rate into a coded data packet. Besides the calculated heart rate, the MCU 17 includes in the coded data packet a user ID code that actually identifies the detection unit 11, a travelling speed of and/or distance covered by the user also determined by the MCU 17 as auxiliary functions, and error correction bits.

The coded data packet is fed to the transmitter 18, via the modulator 19 for modulation thereby before being transmitted by the transmitter 18 as a wireless RF signal at 5.3kHz resonance under the control of the microprocessor 17, for reception by the display unit 13.

The display unit 13 includes an RF receiver 21 for receiving the RF signal, i.e. coded data packet, from the detection unit 11, and its own MCU 22 for control including demodulating and processing the coded data packet to extract the encoded data therefrom, i.e. the calculated heart rate and travelling speed/distance if included, for display on the LCD panel 23.

Figure 3A shows a typical heart rate sequence 30, in that each pulse of the waveform represents a heart beat. A typical RF protocol 31 for a conventional wireless heart rate monitor is shown in Figure 3B, according to which a burst of pulses is transmitted each time a heartbeat is detected. The heart rate is then calculated at the remote display unit, and displayed. As the bursts of pulses are fairly basic in structure, their transmission is vulnerable to interference by other RF sources, and this sometimes leads to incorrect heart rate readings.

With the use of the subject monitoring system 10, heart rate computation is done right at the detection unit 11, and the calculated heart rate is then transmitted by the detection unit 11 as a coded packet of data (i.e. the improved RF protocol of Figure 3C) including a user ID, whereby data integrity is ensured.

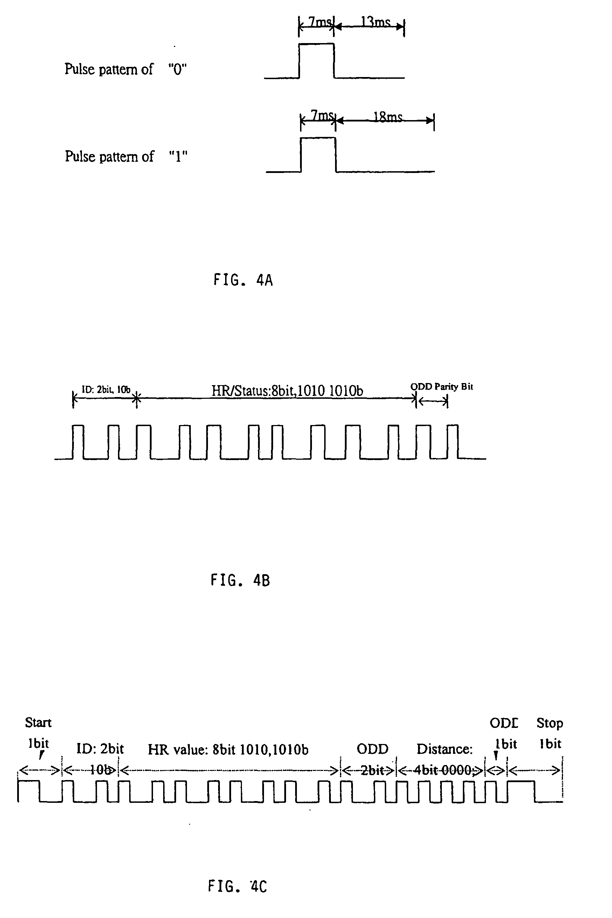

Figure 4A depicts the pulse patterns of "0" and "1", which are distinguished by their length. Figure 4B shows the data format for heart rate information signals generated by the MCU 17 of the detection unit 11 for transmission, which include a 2-bit user ID for user identification (up to four users). Immediately after the 2-bit user ID, there is a 8-bit heart rate reading, which is then followed by a 2-bit ODD parity bit.

Figure 4C shows the data format of generally the same signals for transmission but extended to carry the heart rate information as well as the speed information. The data string begins with a start bit, followed by the same 2-bit user ID, 8-bit heart rate reading and 2-bit ODD parity bit, and then a 4-bit speed/distance information, a 1-bit ODD parity bit and a stop bit.

The coded data packet contains a calculated heart rate value accompanied by a user ID code, and includes error correction bits. Upon receiving a corrupted packet, the wrist-watch receiver 13 will decode the error correction bits and provide a warning of external interference by flashing an icon, for example, on the display 23. The display 23 may hold the current screen until a correct signal is received again, or if interference persists the receiver 13 may invite the user to change location by, for example, dropping the reading to zero.

Heartbeat signals are gathered and computed by the MCU 17 embedded in the chest belt unit 11 attached to the user, and the computed heart rate in beats per minute is then transmitted to the wrist-mounted unit 13 for display. As the ECG pulse gathering and computation both take place right at the chest belt unit 11 that is in close contact with the user's body (heart), interference is minimised. The computed heart rate from the chest belt unit 11 is transmitted as coded data packets in fixed intervals say every two seconds, with information containing the user ID, heart rate in beats per minute, checksum and error guarding bits to ensure that data is transmitted, received and displayed accurately.

The subject wireless heart rate monitoring system 10 supports multiple user operation to avoid signal interference between users in the same vicinity each using a pair of the detection and display units 11 and 13. This is done by time multiplexing the heart rate transmissions from the individual users in different time slots or channels of a series.

The time slots are preferably equal time periods divided from the aforesaid fixed interval (of say two seconds) between successive data packet transmissions, so that different detection units 11 may transmit data at different times, without crashing of data. To cater for four users, each time slot may be half a second long for a detection unit transmission interval of two seconds.

In each detection unit 11, there is a timing circuit or counter, which is preferably embedded in or implemented by the microprocessor 17, for determining one of the time slots for transmission. This timing circuit is associated with a button (or key or any other form of operator) provided on the front of the detection unit 11 for manual operation to change the transmission time slot.

In practice, upon the wrist-watch receiver 13 receiving a corrupted data packet (by reason of interference) as indicated by the flashing icon, the user should press the said button on the detection unit 11 successively to trigger the timing circuit to switch or shift the time of data transmission to the next time slot until an open or vacant time slot is reached (i.e. the interference problem ceases).

As the heart rate is calculated inside the chest belt unit 11, the remote display unit 13 can be a very simple device for merely receiving and displaying data. With the use of a coherent design on the data packet transmission protocol, the display unit 13 may receive and display data from any other categories of data-transmitting devices such as body fat scales, blood pressure meters and pedometers, etc., whereby a generic health care monitoring system is made possible.

It should be appreciated that modifications of and/or alterations to the described embodiment obvious to persons skilled in the art are not to be considered as beyond the scope of the present invention. For example, a bi-directional wireless communication link may be used between the detection and display units. Furthermore, the display unit may be designed to recognise and receive signals from different heartbeat detection units by other manufacturers found on the market, by including the necessary circuitry for data decoding or conversion, etc.

## Claims

1. A wireless heart rate monitoring system (10) comprising:
a detection unit (11) and a display unit (13), the two units being separate and co-operatively in communication via a wireless link (20);
the detection unit comprising:
a casing (12);
attaching means at the casing for attaching the casing on a user;
a heart rate detector (14) at the casing for detecting ECG pulses of said user;
a microprocessor (17) in the casing for computing a heart rate based on the ECG pulses detected by the heart rate detector; and
a transmitter (18) at the casing for transmitting a wireless signal indicative of the heart rate computed by the microprocessor to the display unit;
the display unit comprising:
a casing:
a receiver (21) at the casing for receiving said wireless signal from the detection unit; and
a display (23) at the casing for displaying the heart rate received by the receiver, the detection unit includes timing means for controlling its transmitter to transmit said wireless signal at a different time from that of another detection unit nearby,
**characterised in that** the transmitter is controlled by the microprocessor to transmit said wireless signal at substantially fixed intervals, and the timing means comprises a series of time slots divided from said fixed interval, one of which time slots is allocated for the transmitter to transmit said wireless signal,
and that the timing means is associated with a button or key for manual operation to change the transmission time slot, whereby successive depression of the button or key shifts the time of transmission of said wireless signal from one time slot to the next until a vacant time slot is reached.

2. The monitoring system as claimed in claim 1 **characterised in that** the attaching means comprises a strap or straps for attaching around the chest of said user.

3. The monitoring system as claimed in claim 1, **characterised in that** the display unit is of a wrist-mounted type.

4. The monitoring system as claimed in claim 1, **characterised in that** the transmitter comprises a radio frequency transmitter and the receiver comprises a radio frequency receiver.

5. The monitoring system as claimed in claim 1, **characterised in that** the transmitter is controlled by the microprocessor to transmit said wireless signal at substantially fixed intervals.

6. The monitoring system as claimed in any one of the previous claims, **characterised in that** said wireless signal comprises a coded data packet that includes data representing the heart rate computed by the microprocessor.

7. The monitoring system as claimed in claim 6, **characterised in that** the coded data packet further includes a code identifying the detection unit.

## Patentansprüche

1. Drahtloses Herzfrequenzüberwachungssystem (10), umfassend:
eine Ermittlungseinheit (11) und eine Anzeigeeinheit (13), wobei die beiden Einheiten getrennt sind und zusammenarbeitend in Kommunikation über eine drahtlose Verbindung (20) stehen;
wobei die Ermittlungseinheit umfasst:
ein Gehäuse (12);
Befestigungsmittel am Gehäuse zum Befestigen des Gehäuses an einem Benutzer,
einen Herzfrequenzermittler (14) am Gehäuse zum Ermitteln von EKG-Impulsen des Benutzers;
einen Mikroprozessor (17) im Gehäuse zum Berechnen einer Herzfrequenz auf der Grundlage der durch den Herzfrequenzermittler ermittelten EKG-Impulse; und
einen Sender (18) am Gehäuse zum Übertragen eines drahtlosen Signals, das kennzeichnend für die durch den Mikroprozessor berechnete Herzfrequenz ist, an die Anzeigeeinheit;
wobei die Anzeigeeinheit umfasst:
ein Gehäuse;
einen Empfänger (21) am Gehäuse zum Empfangen des drahtlosen Signals von der Ermittlungseinheit; und
eine Anzeige (23) am Gehäuse zum Anzeigen der durch den Empfänger empfangenen Herzfrequenz, wobei die Ermittlungseinheit Zeitgebermittel zum Steuern ihres Senders aufweist, um das drahtlose Signal zu einem anderen Zeitpunkt als demjenigen einer anderen nahe gelegenen Ermittlungseinheit zu übertragen,
**dadurch gekennzeichnet, dass** der Sender durch den Mikroprozessor gesteuert wird, um das drahtlose Signal in im wesentlichen feststehenden Intervallen zu übertragen, und das Zeitgebermittel eine Folge von aus dem feststehenden Intervall abgeleiteten Zeitschlitzen umfasst, wobei einer der Zeitschlitze dem Sender zugeteilt ist, um das drahtlose Signal zu übertragen,
und **dadurch**, dass das Zeitgebermittel mit einem Knopf oder einer Taste zur manuellen Betätigung verbunden ist, um den Übertragungszeitschlitz zu ändern, wonach fortlaufendes Niederdrücken des Knopfes oder der Taste den Zeitpunkt der Übertragung des drahtlosen Signals von einem Zeitschlitz zum nächsten verschiebt, bis ein unbelegter Zeitschlitz erreicht wird.

2. Überwachungssystem nach Anspruch 1, **dadurch gekennzeichnet, dass** das Befestigungsmittel ein Halteband oder Haltebänder zum Befestigen rings um die Brust des Benutzers umfasst.

3. Überwachungssystem nach Anspruch 1, **dadurch gekennzeichnet, dass** die Anzeigeeinheit von am Handgelenk angebrachter Art ist.

4. Überwachungssystem nach Anspruch 1, **dadurch gekennzeichnet, dass** der Sender einen Funkfrequenzsender umfasst und der Empfänger einen Funkfrequenzempfänger umfasst.

5. Überwachungssystem nach Anspruch 1, **dadurch gekennzeichnet, dass** der Sender durch den Mikroprozessor gesteuert wird, um das drahtlose Signal in im wesentlichen feststehenden Intervallen zu übertragen.

6. Überwachungssystem nach einem der vorhergehenden Ansprüche, **dadurch gekennzeichnet, dass** das drahtlose Signal ein codiertes Datenpaket umfasst, das Daten aufweist, welche die durch den Mikroprozessor berechnete Herzfrequenz darstellen.

7. Überwachungssystem nach Anspruch 6, **dadurch gekennzeichnet, dass** das codierte Datenpaket ferner einen Code aufweist, der die Ermittlungseinheit kenntlich macht:

## Revendications

1. Système de surveillance de fréquence cardiaque sans fil (10) comprenant :
une unité de détection (11) et une unité d'affichage (13), les deux unités étant séparées et en communication coopérative via une liaison sans fil (20) ;
l'unité de détection comprenant :
un boîtier (12) ;
un moyen de fixation au niveau du boîtier pour fixer le boîtier à un utilisateur ;
un détecteur de fréquence cardiaque (14) au niveau du boîtier pour détecter des impulsions ECG dudit utilisateur ;
un microprocesseur (17) dans le boîtier pour calculer la fréquence cardiaque sur la base des impulsions ECG détectées par le détecteur de fréquence cardiaque ; et
un émetteur (18), au niveau du boîtier, pour transmettre un signal sans fil indiquant la fréquence cardiaque calculée par le microprocesseur à l'unité d'affichage ;
l'unité d'affichage comprenant :
un boîtier :
un récepteur (21), au niveau du boîtier, pour recevoir ledit signal sans fil en provenance de l'unité de détection ; et
un affichage (23), au niveau du boîtier, pour afficher la fréquence cardiaque reçue par le récepteur, l'unité de détection comprenant un moyen de synchronisation pour amener son émetteur à transmettre ledit signal sans fil à un instant distinct de celui d'une autre unité de détection à proximité ;
**caractérisé en ce que** l'émetteur est commandé par le microprocesseur pour transmettre ledit signal sans fil à des intervalles sensiblement fixes, et le moyen de synchronisation comprend une série de tranches de temps divisées à partir dudit intervalle fixe, dont l'une des tranches de temps est affectée à l'émetteur pour transmettre ledit signal sans fil ; et
**en ce que** le moyen de synchronisation est associé à un bouton ou une touche en vue d'une opération manuelle destinée à changer la tranche de temps de transmission, moyennant quoi une désactivation successive du bouton ou de la touche décale le temps de transmission dudit signal sans fil, d'une tranche de temps à l'autre, jusqu'à ce qu'une tranche de temps vacante soit atteinte.

2. Système de surveillance selon la revendication 1, **caractérisé en ce que** le moyen de fixation comprend une sangle ou des sangles destinées à être fixées autour de la poitrine dudit utilisateur.

3. Système de surveillance selon la revendication 1, **caractérisé en ce que** l'unité d'affichage est de type fixée au poignet.

4. Système de surveillance selon la revendication 1, **caractérisé en ce que** l'émetteur comporte un émetteur de radiofréquence et le récepteur comporte un récepteur de radiofréquence.

5. Système de surveillance selon la revendication 1, **caractérisé en ce que** l'émetteur est commandé par le microprocesseur afin de transmettre ledit signal sans fil à des intervalles sensiblement fixes.

6. Système de surveillance selon l'une quelconque des revendications précédentes, **caractérisé en ce que** ledit signal sans fil comprend un paquet de données codées lequel inclut des données représentant la fréquence cardiaque calculée par le microprocesseur.

7. Système de surveillance selon la revendication 6, **caractérisé en ce que** le paquet de données codées inclut en outre un code identifiant l'unité de détection.
